# EUROPEAN PATENT APPLICATION

(11) **EP 2 719 363 A1**
(43) Date of publication of application: **16.04.2014**
(21) Application number: 12800985.9
(22) Date of filing: 13.06.2012
(51) Int. Cl.: A61F 5/44, A61G 9/00, A61M 1/00

(54) **DISIMPACTION BAG**

(30) Priority: 13.06.2011 JP 2011131415
(71) Applicant: National University Corporation Nagoya University, Nagoya-shi, Aichi 464-8601 (JP)
(72) Inventor: MAEKAWA, Atsuko, Nagoya-shi Aichi 464-8601 (JP); ONISHI, Kenji, Higashiosaka-shi Osaka 578-0965 (JP)
(74) Representative: Roth, Klaus
(86) International application number: PCT/JP2012/065133
(87) International publication number: WO 2012/173146

(57) **Abstract**

A disimpaction bag includes a bag-shaped main body, an adhesive portion, and at least one finger insertion portion. The at least one finger insertion portion protrudes from either of a lateral surface and a bottom surface of the main body toward inside of the main body, and has a closed tip. The at least one finger insertion portion is configured so as to accommodate at least one finger inserted therein from outside of the main body, and to insert at least one portion thereof into an anus through the opening of the main body.

## Description

### CROSS-REFERENCE TO RELATED APPLICATION

This international application claims the benefit of Japanese Patent Application No. 2011-131415 filed on June 13, 2011 in the Japan Patent Office, and the entire disclosure of Japanese Patent Application No. 2011-131415 is incorporated herein by reference.

### TECHNICAL FIELD

The present invention relates to a disimpaction bag (Disimpaction Bag) that can be used, for example, in a medical procedure called disimpaction for a patient having difficulty in self-defecation.

### BACKGROUND ART

A defecation aid method called disimpaction is a medical procedure performed by a nurse or the like under a doctor's instruction given for a patient having difficulty in discharging stool on his own, and practiced on a daily basis in the work at hospitals/clinics, home care, and nursing facilities for the elderly (see Patent Document 1). Defecation care involves the sense of shame in addition to the risk of anal bleeding and a great mental and physical burden. However, defecation care is an indispensable aid for life support, and therefore disimpaction is necessary for a patient who cannot independently defecate due to a passage disturbance in the lower digestive tract or intestinal paralysis.

One example of a disimpaction method is performed such that an aide puts on latex gloves, inserts fingers into the anus so as to crumble and then take out a lump of stool accumulated inside of the rectum/colon, and wipe off the stool with disposable diapers or drop the stool into a portable toilet.

### PRIOR ART DOCUMENT

### PATENT DOCUMENT

Patent Document 1: Japanese Unexamined Patent Application Publication No. 2003-210571

### SUMMARY OF THE INVENTION

### PROBLEMS TO BE SOLVED BY THE INVENTION

In the above-described example, the odor from accumulated stool gives an unpleasant feeling to both the patient and the nurse. Moreover, in the above-described example, the risks of transmission or droplet infection exist.

It is preferable that one aspect of the present invention can provide a disimpaction bag that enables reducing the stool odor, contact with stool, or the risk of droplet infection.

### MEANS FOR SOLVING THE PROBLEMS

A disimpaction bag according to the present invention includes: a bag-shaped main body that stores stool therein, the main body being provided with an opening at one end thereof, and made of a deformable material; an adhesive portion that causes to adhesively attach the disimpaction bag to skin around an anus, the adhesive portion being disposed along the opening and having adhesiveness; and at least one finger insertion portion, protruding from either of a lateral surface and a bottom surface of the main body toward inside of the main body, and having a closed tip. The at least one finger insertion portion is configured so as to accommodate at least one finger inserted therein from outside of the main body, and to insert at least one portion thereof into the anus through the opening.

The disimpaction bag according to the present invention can be used as follows: the disimpaction bag is, first of all, attached to a patient such that the adhesive portion of the disimpaction bag is adhesively attached to skin around the anus of the patient. At this time, the opening of the disimpaction bag is faced to the anus of the patient, and can be contacted with, or preferably tightly attached to a surrounding portion of the anus. Moreover, the inside of the disimpaction bag is preferably sealed from exterior environment. Subsequently, while at least one finger (for example, index finger and middle finger) of a nurse or the like is inserted from outside of the main body into the at least one finger insertion portion, at least one portion of the at least one finger insertion portion is inserted into the anus and furthermore into the rectum. Then, the nurse or the like moves the at least one finger, inserted into the at least one finger insertion portion, so as to take out stool from the anus of the patient. The manually discharged stool is dropped into the main body. When disimpaction is over, the disimpaction bag is removed from the skin around the anus of the patient, and discarded.

Moreover, the adhesive portion may be provided with fold portions so as to fold the adhesive portion in half. In this case, by using the fold portions and folding the adhesive portion in half, the opening of the main body can be easily closed. In a case wherein the opening of the main body is closed, spreading of stool odor or infection can be inhibited.

Since the disimpaction bag according to the present invention can store manually discharged stool in the bag-shaped main body and the main body can be tightly sealed, stool odor can be inhibited from spreading. Moreover, since the odor does not easily spread, psychological burdens on patients can be reduced.

Patients can move their bodies and change positions while disimpaction bags are attached. As a result, for example, if a disimpaction bag is attached after a suppository being inserted or an enema being given for stimulating bowel movement, an anal region can be held so that enema liquid does not leak from the top of the bag. Patients can maintain comfortable postures until the defecation reflex takes place and wait until stool exits the body. After the defecation reflex takes place, the enema liquid and stool can directly be stored in the disimpaction bag. When disimpaction treatment follows immediately after, the patient can receive the treatment without any concern for odor.

Since the disimpaction bag according to the present invention stores stool therein, nurses or the like do not need to directly touch the stool. As a result, even if stool contains infectious microorganisms or viruses such as for MRSA, hepatitis, O-157, AIDS, and so on, infection to nurses or the like can be inhibited. In addition, even if a patient receives anticancer drug treatment and his stool contains toxic chemicals, exposure to nurses or caregivers can be inhibited.

The at least one finger insertion portion may be a plurality of finger insertion portions. In this case, each of the plurality of finger insertion portions may be formed in a position so that distance from each of the plurality of finger insertion portions to the opening is equivalent to each other.

Unlike in conventional methods, using the disimpaction bag according to the present invention dispenses necessity for separately preparing disposable diapers, lubricant, deodorizer, and so on.

In a case wherein the at least one finger insertion portion is a plurality of finger insertion portions, each of the plurality of finger insertion portions may be formed in a position so that distance from each of the plurality of finger insertion portions to the opening is different from each other. Respectively disposing the plurality of finger insertion portions in such positions facilitates operation with the plurality of fingers inserted into the plurality of finger insertion portions, and makes the treatment of disimpaction much easier. For example, while an index finger and a middle finger are respectively inserted from outside of the main body into two finger insertion portions, at least some portions of the two finger insertion portions are inserted into the anus and furthermore into the rectum, and the index finger and the middles finger are both moved, stool can be more easily taken out.

The adhesive portion may be an oval sheet-like member having a hole in a center thereof, and be provided with a tab in the outer periphery thereof. Having the tab enables weighing the disimpaction bag containing stool by a spring scale.

Lubricant may be applied at least to a portion of an inner surface of the main body that forms the at least one finger insertion portion and to a surrounding area. Application of the lubricant enables smoothly taking out stool with fingers without giving any damage to the anus and surrounding mucous membrane. The lubricant may be applied to an entire portion of the inner surface of the main body, or only on the portion that forms the at least one finger insertion portion.

The deformable material is not particularly limited. For example, various types of rubber, plastic (particularly plastic having stretchability), paper, paper with resin coating on a surface, and so on can be used. The material may also have stretchability. Moreover, the number of the at least one finger insertion portion may preferably be two so that an index finger and a middle finger can be respectively inserted, however may be any other numbers.

### BRIEF DESCRIPTION OF THE DRAWINGS

Fig. 1 is a perspective view showing a structure of a disimpaction bag according to a first embodiment.
Figs. 2A-2D are explanatory views showing a manufacturing process of the disimpaction bag according to the first embodiment.
Fig. 3 is an explanatory view showing a usage method of the disimpaction bag according to the first embodiment.
Fig. 4 is a perspective view showing a variation of the disimpaction bag in an enlarged manner.
Fig. 5 is an explanatory view showing a positional relation of two finger insertion portions, a tab, and fold portions in a top view of the disimpaction bag according to the first embodiment.
Fig. 6 is a perspective view showing a structure of a disimpaction bag according to a second embodiment.
Fig. 7 is an explanatory view showing a positional relation of two finger insertion portions, a tab, and fold portions in a top view of the disimpaction bag according to the second embodiment.
Fig. 8 is a sectional side view showing a structure of a disimpaction bag according to a third embodiment.
Fig. 9 is a perspective view showing a structure of a disimpaction bag according to a fourth embodiment.
Fig. 10 is an explanatory view showing one part of a manufacturing process of the disimpaction bag according to the fourth embodiment.
Fig. 11 is an explanatory view showing an example of a usage method of the disimpaction bag according to the fourth embodiment.

### EXPLANATION OF REFERENCE NUMERALS

1... disimpaction bag, 3,20...main body, 6...adhesive portion, 5a...skin attachment surface, 5a...fold portions, 7...opening, 9,11...finger insertion portions, 12...hole, 13...tab, 13a...hole, 15,17...convex portions, 19...end portion, 21... adhesive portion, 23...adhesive layer, 101...patient, 103...anus

### MODE FOR CARRYING OUT THE INVENTION

Embodiments according to the present invention will be explained based on the drawings.

### <First Embodiment>

### 1. Structure of disimpaction bag 1

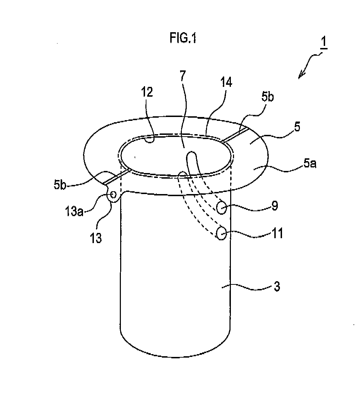

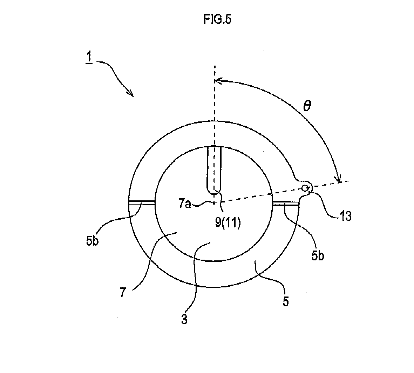

The structure of a disimpaction bag 1 is now described based on Figs. 1 and 5. The disimpaction bag 1 includes a bag-shaped main body 3, made of a thin rubber membrane, which is low-allergy, transparent, and latex·free (a reformable and stretchable material), and an adhesive portion 5. The bag-shaped main body 3 stores manually discharged stool, mucous membrane, and the like. It is to be noted that the disimpaction bag 1 may be translucent.

The main body 3 includes an opening 7 at one end thereof. The shape of the opening 7 may be circular or oval. The capacity of the main body 3 is 1000ml. On the lateral surface of the main body 3 (the portion that becomes the lateral surface when the opening 7 faces upward, as shown in Fig. 1), finger insertion portions 9, 11 are respectively formed in concave manners so that hand fingers can be inserted therein. The finger insertion portions 9, 11 are integrally configured with other portions of the main body 3, and made of a thin rubber membrane that is transparent and latex-free. That is, the finger insertion portions 9, 11 are concave portions formed by a portion of the main body 3 being inwardly introduced.

When the disimpaction bag 1 is placed as shown in Fig. 1, the finger insertion portion 9 is disposed in the upper side of the finger insertion portion 11. That is, the finger insertion portion 9 and the finger insertion portion 11 are disposed along the vertical line in Fig. 1. The finger insertion portion 9 has a shorter distance to the opening 7 as compared with the finger insertion portion 11. The interval between the finger insertion portion 9 and the finger insertion portion 11 is determined such that a nurse or the like can easily insert his index finger into the finger insertion portion 9 and middle finger into the finger insertion portion 11. Since the tips of the finger insertion portion 9 and the finger insertion portion 11 are closed, when a nurse or the like inserts his fingers into an intestinal tract while the fingers are inserted into the finger insertion portions 9, 11 (for example 5-7cm) from outside of the main body 3, the tips of the fingers do not directly touch stool and the like.

On the inner surface of the main body 3, water·soluble lubricant is applied. This water-soluble lubricant is also applied to the portions that form the finger insertion portions 9, 11. For the water-soluble lubricant, water-soluble lubricating jelly may be used.

The above-mentioned adhesive portion 5 is an oval sheet-like member having a hole 12 formed in the central portion thereof. The adhesive portion 5 is attached to the opening 7 of the main body 3. More specifically, an adhesion area 14, provided on the bottom surface (the bottom surface shown in Fig. 1) of the adhesive portion 5, and an end portion of the main body 3, provided in the side of the opening 7, are adhered. The adhesion area 14 extends along the entire circumference of the hole 12. The entire part of the end portion of the main body 3 in the side of the opening 7 is adhesively attached to the adhesive portion 5. Since the adhesive portion 5 and the main body 3 are adhered as explained above, the hole 12 of the adhesive portion 5 faces the opening 7. Inside of the main body 3 is sealed from the exterior environment except in the region of the hole 12 of the adhesive portion 5. Moreover, the adhesive portion 5 extends more outwardly than the main body 3.

On a skin attachment surface 5a of the adhesive portion 5, provided in a surface opposite to the main body 3 (the upper surface in Fig. 1), an adhesive layer is formed so that the skin attachment surface 5a can be adhesively attached to skin. Moreover, the adhesive portion 5 is made of a hydrocolloid (Hydro-colloid) skin protective material that gives fewer burdens on skin even when the adhesive portion 5 is adhesively attached to skin. In one portion of the outer circumference of the adhesive portion 5, a tab 13 is formed. The central portion of the tab 13 is provided with a hole 13a. As described above, the adhesive portion 5, provided along the opening 7 of the main body 3 and having adhesiveness, is configured such that the adhesive portion 5 can be tightly attached to skin around the anus of a patient.

On the skin attachment surface 5a, fold portions 5b are formed. The fold portions 5b are grooves that are one step depressed from the surrounding portion in the skin attachment surface 5a. Each of the fold portions 5b is disposed on a fold line, provided in order to evenly fold the adhesive portion 5 in half (that is, on a straight line that halves the adhesive portion 5). The adhesive portion 5 can be folded in half along the fold line 5b such that the skin attachment surface 5a is placed inside of the fold. When the adhesive portion 5 is folded in half, the hole 12 of the adhesive portion 5 and the opening 7 of the main body 3 are closed. Moreover, when the adhesive portion 5 is folded in half, the adhesive layer of the skin attachment surface 5a in both sides is adhered and maintains the double folded state.

When the disimpaction bag 1 is viewed from the upper side (the upper side in Fig. 1), and a circle whose center point is the center point 7a of the opening 7 is assumed, on the circumference of the circle, the tab 13 is disposed, as shown in Fig. 5, in a position which is θ rotation away in the clockwise direction from the positions of the finger insertion portions 9, 11. The value of θ is in the rage of 45-135 degrees. Moreover, one of the fold portions 5b is disposed in the vicinity of the tab 13, and the other fold portion 5b is disposed on the opposite side on the circumference.

### 2. Manufacturing method of the disimpaction bag 1

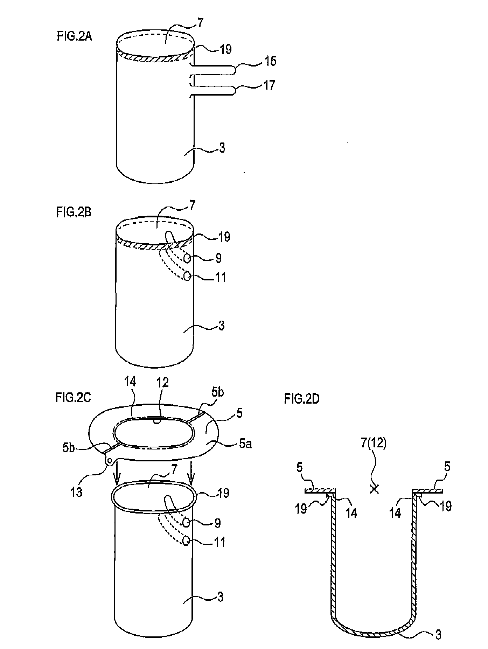

The method of manufacturing the disimpaction bag 1 is now explained based on Figs. 2A-2D. First, as shown in Fig. 2A, the bag-shaped main body 3, provided with the opening 7, is formed. To the main body 3, two finger insertion portions are provided (which, in this case, are formed as convex portions 15, 17 protruding from inside of the main body 3 toward outside of the main body 3). It is to be noted that the convex portions 15, 17, formed so as to become finger insertion portions, are integrally formed with other portions of the main body 3, and have similar material quality and thickness with respect to other portions of the main body 3.

Subsequently, as shown in Fig. 2B, the finger insertion portions (in this case, formed as the convex portions 15, 17) are pushed inside of the main body 3. As a result, the finger insertion portions 9, 11 are formed into which fingers can be inserted from outside of the main body 3 (in this case, the convex portions 15, 17 are pushed inside of the main body 3 and become concave portions). In this case, the finger insertion portions 9, 11, having closed tips, are formed so as to protrude from the lateral surface of the main body 3 toward inside of the main body 3. The finger insertion portions 9, 11 have a certain length and degree of resilience so that at least some portions of the finger insertion portion 9, 11 (some portions including the tips) can be inserted into the anus of a patient through the opening 7.

Subsequently, an end portion 19 (see Figs. 2A and 2B) of the main body 3 in the side of the opening 7 is folded, as shown in Fig. 2C, so as to outwardly extend. Then, to the end portion 19, the adhesion area 14 of the adhesive portion 5 is adhesively attached. The attachment may be done by using heat welding. A sectional side view of the above-described attached state of the main body 3 and the adhesive portion 5 is shown in Fig. 2D.

Next, to the inner surface of the main body 3, the water-soluble lubricant is applied. In addition, release paper, which is not shown in the drawing, is attached to the skin attachment surface 5a of the adhesive portion 5. It is to be noted that this release paper is removed when the disimpaction bag 1 is used. The disimpaction bag 1, manufactured as above, can be individually packed.

### 3. Method for using disimpaction bag 1

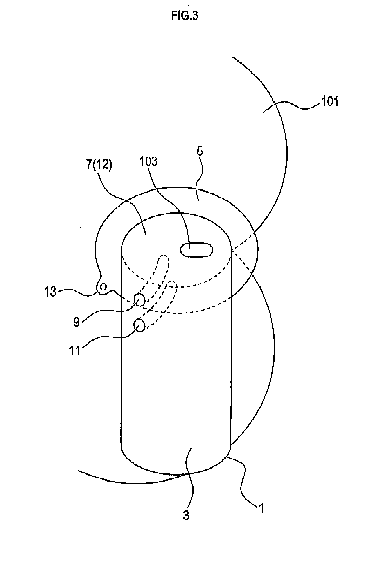

The method of using the disimpaction bag 1 is now explained based on Fig. 3. First, the disimpaction bag 1 is taken out from individual packing, and the release paper, adhered to the skin attachment surface 5a of the adhesive portion 5, is removed. Then, the disimpaction bag 1 is attached to a patient 101. The disimpaction bag 1 is attached such that the skin attachment surface 5a adheres around the anus 103 of the patient 101. At this time, the opening 7 of the disimpaction bag 1 faces the anus 103. Moreover, the inside of the disimpaction bag 1 is sealed from the exterior environment.

After the disimpaction bag 1 is attached, an index finger of a nurse or the like is inserted into the finger insertion portion 9, and a middle finger is inserted into the finger insertion portion 11. The finger insertion portions 9, 11, wherein the fingers of the nurse or the like are inserted, and the inner surface of the main body 3 are stretched toward the opening 7 so that at least some portions of the finger insertion portions 9, 11 (for example, some portions including the tips thereof) are inserted into the anus 103 and moved therein in order to take out stool from the anus of the patient 101. The manually discharged stool is dropped into the main body 3. When the disimpaction is over, the disimpaction bag 1 is removed from the patient and discarded. When the disimpaction bag 1 is discarded, the adhesive portion 5 is preferably folded in half so that the both sides of the skin attachment surface 5a are adhered to each other. As a result, the opening 7 is closed, and spreading of stool odor or infection can be inhibited. Moreover, after the disimpaction bag 1 is removed from the patient 101, the tab 13 can be placed on a hook of a spring scale so as to weigh the disimpaction bag 1. If the weight of the disimpaction bag 1 alone is measured in advance, by subtracting the bag weight from the weight of the disimpaction bag 1 with stool being contained therein, the weight of the stool alone can be calculated.

### The effect of disimpaction bag 1

(1) The disimpaction bag 1 can store manually discharged stool therein and can be tightly sealed. As a result, stool odor can be inhibited from spreading. Moreover, since the odor does not easily spread, a psychological burden on a patient can be reduced. It is to be noted that the sealing of the disimpaction bag 1 can be easily realized by folding the adhesive portion 5 in half on the fold portions 5b such that the inner side of the skin attachment surface 5a is placed inside of the fold. In this case, since the adhesive layer of the skin attachment surface 5a in both sides is adhered to each other, the folded state (sealed state of the disimpaction bag 1) can be maintained. If the disimpaction bag 1 is transparent or translucent, stool condition can be easily observed.
(2) Patients can move their bodies and change positions while disimpaction bags are attached. As a result, for example, if a disimpaction bag is attached after a suppository being inserted or an enema being given for stimulating bowel movement, an anal region can be held so that enema liquid does not leak from the top of the bag. A patient can maintain a comfortable posture until the defecation reflex takes place, and wait until stool exits the body. After the defecation reflex takes place, the enema liquid and stool can be directly stored in the disimpaction bag. When disimpaction treatment follows immediately after, the patient can receive the treatment without any concern for the odor.
(3) Since the disimpaction bag 1 stores stool, blood, mucous membrane, and so on therein, these stool, blood, mucous membrane and so on do not contact with nurses or the like. As a result, even if stool contains infectious microorganisms or viruses such as for MRSA, hepatitis, O-157, AIDS, infection to nurses or the like can be inhibited. In addition, even if a patient receives an anticancer drug treatment and his stool contains toxic chemicals, exposure thereof to nurses or the like can be inhibited.
(4) The disimpaction bag 1 is configured in an all-in-one manner and includes all the necessary components for disimpaction. Therefore, disposable diapers, lubricant, deodorizer, plastic bags, and so on do not have to be separately prepared.
(5) Using the disimpaction bag 1 enables easily weighing the amount of stool.
(6) Application of the water-soluble lubricant to the inner surface of the main body 3 enables smoothly taking out stool with fingers without giving any damage to anus and the surrounding mucous membrane.
(7) By arranging the positional relation of the tab 13 and the finger insertion portions 9, 11 as shown in Fig. 5, the disimpaction bag 1 can be easily attached in an appropriate direction. That is, to a patient in a recumbent position, if the disimpaction bag 1 is attached in a way so that the tab 13, which can be a mark, is directed upward, the finger insertion portions 9, 11 are disposed in some positions that make the work easy for a right-handed nurse or the like.

### <Second Embodiment>

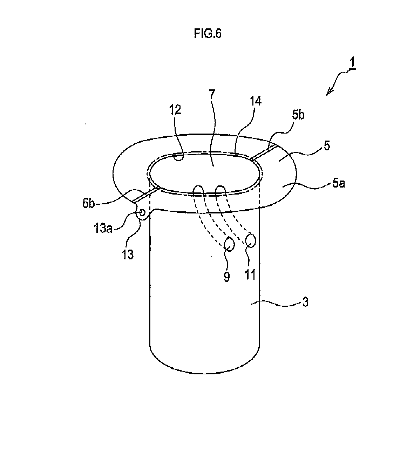

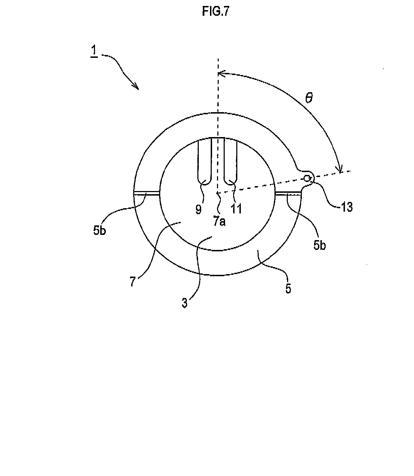

The structure of a disimpaction bag 1 according to the present embodiment is now explained based on Figs. 6 and 7. The structure of the disimpaction bag 1 according to the present embodiment is basically similar to the structure in the above-described first embodiment. The difference is that the distance from the finger insertion portion 9 to the opening 7 and the distance from the finger insertion portion 11 to the opening 7 are equivalent. That is, the positional relation of the finger insertion portions 9, 11 is, as shown in Fig. 6, such that when the disimpaction bag 1 is placed with the opening 7 facing upward, the finger insertion portions 9, 11 are laterally aligned with a predetermined interval therebetween. The interval between the finger insertion portion 9 and the finger insertion portion 11 is determined such that nurses or the like can easily insert their index fingers into one of the finger insertion portions 9, 11 and insert their middle fingers into the other finger insertion portion.

When the disimpaction bag 1 is viewed from the upper side (the upper side in Fig. 6), and a circle whose center point is the center point 7a of the opening 7 is assumed, on the circumference of the circle, the tab 13 is disposed, as shown in Fig. 7, in a position which is θ rotation away in the clockwise direction from the central position between the finger insertion portions 9 and 11. The value of θ is in the range of 45-135 degrees. Moreover, one of the fold portions 5b is disposed in the vicinity of the tab 13, and the other fold portion 5b is disposed on the opposite side on the circumference.

The disimpaction bag 1 according to the present embodiment achieves an almost similar effect with respect to the above-described first embodiment.

### <Third Embodiment>

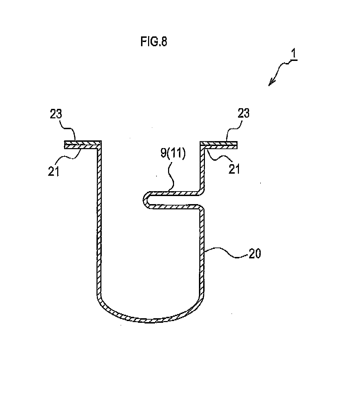

The structure of a disimpaction bag 1 according to the present embodiment is now explained based on Fig. 8. The structure of the disimpaction bag 1 according to the present embodiment is basically similar to the structure in the above-described second embodiment. The difference is that the main body 3 and the adhesive portion 5 are integrally formed. That is, the disimpaction bag 1 according to the present embodiment includes an integrated type of main body 20 having a similar configuration to the combination of the main body 3 and the adhesive portion 5 according to the second embodiment. This main body 20 is a bag-shaped member made of a thin rubber membrane which is low-allergy, transparent, and latex-free (a deformable and stretchable material). The portion of the main body 20, which corresponds to the adhesive portion 5 according to the above-described second embodiment, is an adhesive portion 21 outwardly extending. On the upper surface, shown in Fig. 8, of the adhesive portion 21, adhesive layer 23 is formed which can be adhesively attached to skin. On the main body 20, the finger insertion portions 9, 11 are formed.

The disimpaction bag 1 according to the present embodiment can achieve an almost similar effect with respect to the above-described second embodiment.

### <Fourth Embodiment>

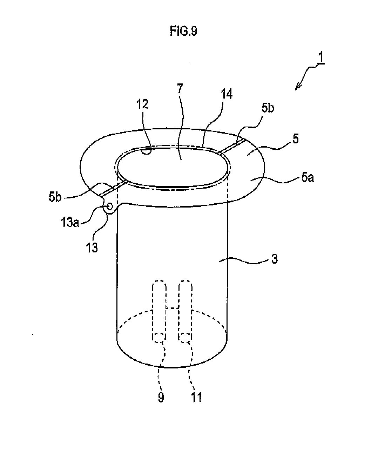

The structure of a disimpaction bag 1 according to the present embodiment is now explained based on Figs. 9-11. The structure of the disimpaction bag 1 according to the present embodiment is basically similar to the above-described first embodiment. The differences are that, as shown in Fig. 9, the finger insertion portions 9, 11 are formed on the bottom surface (a portion facing the opening 7) of the main body 3, and that the main body 3 is made of a polyethylene·based material (deformable and stretchable material). Similarly in the present embodiment, the interval between the finger insertion portion 9 and the finger insertion portion 11 is determined such that a nurse or the like can easily insert his index finger and middle finger.

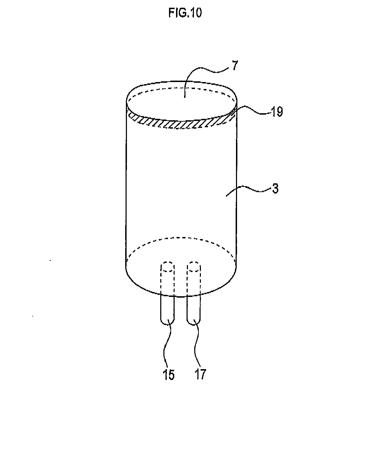

The disimpaction bag 1 is manufactured, similarly to the above-described first embodiment, by adhering the main body 3 and the adhesive portion 5. In the manufacturing of the main body 3, first, as shown in Fig. 10, the bag-shaped main body 3, including outwardly·protruding convex portions 15, 17, is made. The convex portions 15, 17 are integrally formed with other portions of the main body 3, and have similar material quality and thickness with respect to the other portions of the main body 3. Subsequently, the convex portions 15, 17 are pushed inside of the main body 3, as a result of which the finger insertion portions 9, 11, as shown in Fig. 9, are formed.

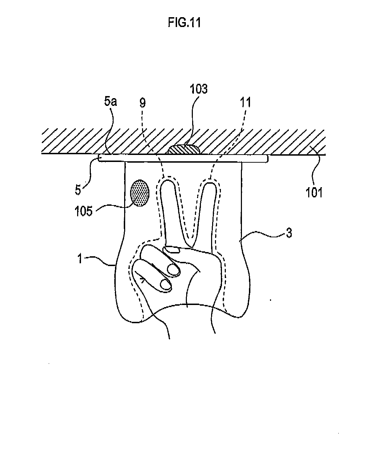

The disimpaction bag 1 is attached to a patient 11 as shown in Fig. 11. The disimpaction bag 1 is attached such that the skin attachment surface 5a of the disimpaction bag 1 is adhesively attached around the anus 103 of the patient 101. At this time, the finger insertion portions 9, 11 of the disimpaction bag 1 face the anus 103. Moreover, the inside of the disimpaction bag 1 is sealed from exterior environment.

After the disimpaction bag 1 is attached, an index finger of a nurse or the like is inserted into the finger insertion portion 11, and a middle finger is inserted into the finger insertion portion 9. The finger insertion portions 9, 11, into which the fingers of the nurse or the like are inserted, and the inner surface of the main body 3 are stretched toward the opening 7 so that at least some portions of the finger insertion portions 9, 11 (for example, some portions including the tips thereof) are inserted into the anus 103 and moved therein in order to take out stool 105 from the anus of the patient 101. In this case, if the main body 3 is made of a polyethylene-based material, which is deformable and stretchable, the above-described work can be easily done. The manually discharged stool 105 is dropped inside of the main body 3.

After disimpaction is over, the disimpaction bag 1 is removed from the patient, and discarded. When the disimpaction bag 1 is discarded, the adhesive portion 5 is preferably folded in half so that the both sides of the skin attachment surface 5a are adhered to each other. As a result, the opening 7 is closed, and spreading of stool odor or infection can be inhibited.

It is to be noted that the disimpaction bag 1 according to the present embodiment may be provided with an integrated main body, which includes a portion corresponding to the adhesive portion 5, similarly to the above-described third embodiment.

It goes without saying that the present invention is not limited to the above-described embodiments, and can be carried out in various ways within the range without departing from the present invention.

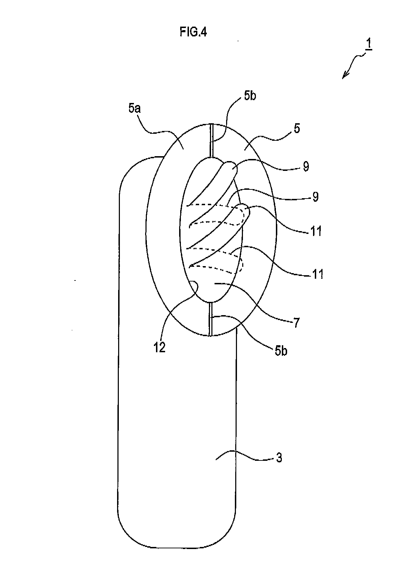

For example, the positions and the number of the finger insertion portion 9, the finger insertion portion 11 are not limited to the positions in the above-described embodiments, but can be arbitrarily determined. For instance, the positions of the finger insertion portions may be changed for right·handed users and for left-handed users. Moreover, the configuration of the disimpaction bag may be changed such that the positional relation of the main body 3 and the adhesive portion 5 shown in Fig. 1 is altered to the positional relation shown in Fig. 4 (variation).

That is, the opening 7 is provided in a lateral position of the main body 3 and the adhesive portion 5 is provided in the periphery of the opening 7. In the case of this variation, a nurse or the like inserts at least some portions of the finger insertion portions 9, 11, into which his fingers are inserted from outside of the main body 3, into the anus 103 of the patient, and performs disimpaction. Since the longitudinal direction of the finger insertion portions 9, 11 become nearly vertical with respect to the opening 7, the insertion into the anus 103 becomes easy. In this case, the tips of the finger insertion portions 9, 11 can be moved to the positions shown with a full line in Fig. 4 and also to the positions shown with the dotted line in Fig. 4.

It is to be noted that the adhesive portion 5 is provided with two fold portions 5b extending in the up·and·down direction so as to fold the adhesive portion 5 in half. The fold portions 5b are grooves that are one step depressed from the surrounding portion on the surface of the adhesive portion 5 (the surface where an adhesive agent exists). Each of the fold portions 5b is disposed on a fold line that evenly divides the adhesive portion 5 in half (that is, a straight line that halves the adhesive portion 5). The adhesive portion 5 can be folded in half on the fold line 5b so that the surface, on which the adhesive agent exists, is placed inside of the fold. When the adhesive portion 5 is folded in half, the opening 7 of the main body 3 is closed. As a result, spreading of stool odor and infection can be inhibited.

## Claims

1. A disimpaction bag comprising:
a bag-shaped main body that stores stool therein, the main body being provided with an opening at one end thereof, and made of a deformable material;
an adhesive portion that causes to adhesively attach the disimpaction bag to skin around an anus, the adhesive portion being disposed along the opening and having adhesiveness; and
at least one finger insertion portion, protruding from either of a lateral surface and a bottom surface of the main body toward inside of the main body, and having a closed tip
wherein the at least one finger insertion portion is configured so as to accommodate at least one finger inserted therein from outside of the main body, and to insert at least one portion thereof into the anus through the opening.

2. The disimpaction bag according to claim 1,
wherein the at least one finger insertion portion is a plurality of finger insertion portions, and
wherein each of the plurality of finger insertion portions is formed in a position so that distance from each of the plurality of finger insertion portions to the opening is different from each other.

3. The disimpaction bag according to claim 1,
wherein the at least one finger insertion portion is a plurality of finger insertion portions, and
wherein each of the plurality of finger insertion portions is formed in a position so that a distance from each of the plurality of finger insertion portions to the opening is equivalent to each other.

4. The disimpaction bag according to one of claims 1 to 3 wherein the adhesive portion is provided with fold portions so as to fold the adhesive portion in half.

5. The disimpaction bag according to one of claims 1 to 4 wherein lubricant is applied at least to a portion of an inner surface of the main body that forms the at least one finger insertion portion.
